(19)

**Europäisches Patentamt**
**European Patent Office**
**Office européen des brevets**

(11) **EP 4 462 357 A1**

(12) **EUROPEAN PATENT APPLICATION**

(43) Date of publication:
**13.11.2024   Bulletin 2024/46**

(21) Application number: **24168450.5**

(22) Date of filing: **04.04.2024**

(51) International Patent Classification (IPC):
**G06T 7/00** (2017.01)          **A61B 3/117** (2006.01)

(52) Cooperative Patent Classification (CPC):
**G06T 7/0012; A61B 3/0025; A61B 3/1176;**
**G06T 7/136; G06T 2207/30041**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **20.04.2023   JP 2023069351**

(71) Applicants:
• **KI MEDIC CO., INC.**
**Atsuta-ku**
**Nagoya-shi, Aichi 456-0032 (JP)**
• **SHINSHU UNIVERSITY**
**Matsumoto City, Nagano, 390-8621 (JP)**

(72) Inventors:
• **Ichikawa, Kazuo**
**Nagoya-shi, Aichi, 456-0032 (JP)**
• **Tanaka, Kiyoshi**
**Nagano City, Nagano, 380-8553 (JP)**
• **Yokoyama, Sho**
**Nagoya-shi, Aichi, 456-0032 (JP)**
• **Tanaka, Yoshiki**
**Nagoya-shi, Aichi, 456-0032 (JP)**
• **Yasuzawa, Kosuke**
**Nagano City, Nagano, 380-8553 (JP)**

(74) Representative: **Ter Meer Steinmeister & Partner**
**Patentanwälte mbB**
**Nymphenburger Straße 4**
**80335 München (DE)**

(54) **IMAGE PROCESSING DEVICE, IMAGE PROCESSING METHOD, AND PROGRAM**

(57)     Provided is an image processing device, an image processing method, or a program that can obtain an image for lens opacity determination that facilitates opacity determination for a lens. An image processing device 4 acquires a transillumination image of a lens captured by a capturing device 2, and performs a flat correction process to reduce illumination unevenness in the transillumination image. In the flat correction process, an average filter is generated on the basis of an original image of the transillumination image, the original image is divided by the average filter, and values after division are multiplied by an average luminance of the original image. The transillumination image after the flat correction process is converted to a binarized image composed of white and black. Determination for lens opacity is performed on the basis of the binarized image.

FIG.2

**Description**

BACKGROUND OF THE INVENTION

Field of the Invention

The present disclosure relates to an image processing device, an image processing method, and a program that process a transillumination image of a lens.

Description of Related Art

As an examination for a cataract, there is an examination in which presence/absence or the degree of an opacity of a lens is determined on the basis of a transillumination image of the lens (see, for example, Mai Kita, "Indication of Cataract Surgery Considering the Age of Patients", The Journal of The Japanese Society for Cataract Research, 2017, Vol. 29, pp. 56-61).

SUMMARY OF THE INVENTION

Conventionally, opacity determination for a lens is performed through subjective assessment by a doctor or the like, and therefore there is a problem that assessment (opacity determination) differs depending on the assessment person's experience or the like.

The present disclosure has been made in view of the above problem, and an object of the present disclosure is to provide an image processing device, an image processing method, or a program that can obtain an image for lens opacity determination that facilitates opacity determination for a lens.

An image processing device according to the present disclosure includes: an acquisition unit configured to acquire a transillumination image including a lens area and an area therearound, the transillumination image having such a luminance gradient that a luminance gradually decreases outward from a center indicating a local-maximum luminance; an extraction unit configured to extract the lens area in the transillumination image; a correction unit configured to perform correction so as to reduce the luminance gradient which is illumination unevenness in an image of the lens area extracted by the extraction unit; and a binarization processing unit configured to binarize pixel values of the corrected image, to obtain a binarized image for lens opacity determination.

An image processing method according to the present disclosure includes: an acquisition step of acquiring a transillumination image including a lens area and an area therearound, the transillumination image having such a luminance gradient that a luminance gradually decreases outward from a center indicating a local-maximum luminance; an extraction step of extracting the lens area in the transillumination image; a correction step of performing correction so as to reduce the luminance gradient which is illumination unevenness in an image of the lens area extracted in the extraction step; and a binarization processing step of binarizing pixel values of the corrected image, to obtain a binarized image for lens opacity determination.

A program according to the present disclosure is configured to cause a computer to execute: an acquisition step of acquiring a transillumination image including a lens area and an area therearound, the transillumination image having such a luminance gradient that a luminance gradually decreases outward from a center indicating a local-maximum luminance; an extraction step of extracting the lens area in the transillumination image; a correction step of performing correction so as to reduce the luminance gradient which is illumination unevenness in an image of the lens area extracted in the extraction step; and a binarization processing step of binarizing pixel values of the corrected image, to obtain a binarized image for lens opacity determination.

With the image processing device, the image processing method, or the program according to the present disclosure, illumination unevenness in a transillumination image is reduced and then pixel values of the transillumination image are binarized, whereby an image for lens opacity determination that facilitates opacity determination for a lens can be obtained.

BRIEF DESCRIPTION OF THE DRAWINGS

FIG. 1 is a configuration diagram of an image processing system;
FIG. 2 is a flowchart of a lens opacity determination process executed by a CPU of an image processing device;
FIG. 3 is a flowchart of a process subsequent to the process shown in FIG. 2;
FIG. 4 shows an example of an original image of a transillumination image of a lens;

FIG. 5 shows an image after a median filter has been applied to the image in FIG. 4;

FIG. 6 shows a binarized image in which a lens area is represented by white and the other area is represented by black;

FIG. 7 is a lens mask image after an extra black area has been cut away from the image in FIG. 6;

FIG. 8 is an example of a transillumination image of a lens area extracted in step S3 in FIG. 2 before flat correction;

FIG. 9 shows luminance changes in the images before and after a flat correction process, with respect to X coordinates;

FIG. 10 is a flowchart showing the details of the flat correction process in step S4 in FIG. 2;

FIG. 11 illustrates a method for generating an average filter and shows pixel values of an original image and squares of the average filter, where a value is partially specified among the squares;

FIG. 12 illustrates the method for generating the average filter and shows the pixel values of the original image and the squares of the average filter, where a value is partially specified among the squares subsequently to FIG. 11;

FIG. 13 shows operation of dividing the pixel values of the original image by the average filter;

FIG. 14 shows operation of multiplying quotient values between the pixel values of the original image and the average filter by an average luminance of the original image;

FIG. 15 is an example of a lens transillumination image after the flat correction process;

FIG. 16 shows examples of a transillumination image after the flat correction process and a binarized image of the transillumination image;

FIG. 17 is an image obtained through processing of exclusive disjunction between the right image in FIG. 16 and the mask image in FIG. 7;

FIG. 18 shows a central area and a peripheral area of a pupil (lens);

FIG. 19 is a mask image of the pupil central area;

FIG. 20 is an image obtained by extracting opacity in the pupil central area;

FIG. 21 illustrates an algorithm of detection for a cortical opacity and shows a center and an approximation line of a target opacity area in a binarized image of a lens opacity;

FIG. 22 is a flowchart showing the details of a cortical opacity extraction process in step S11 in FIG. 3;

FIG. 23 is a mask image of the peripheral area of the pupil (lens);

FIG. 24 shows an example of an opacity area corresponding to a cortical opacity;

FIG. 25 shows an example of an opacity area not corresponding to a cortical opacity;

FIG. 26 is a flowchart showing the details of a cortex assessment process in step S12 in FIG. 3;

FIG. 27 shows an example of an image of an extracted cortical opacity;

FIG. 28 shows a scene of calculating an angle of the cortical opacity;

FIG. 29 is a flowchart showing the details of a posterior subcapsular opacity extraction process in step S13 in FIG. 3;

FIG. 30 shows an example of an opacity image obtained in step S5 and a scene of calculating the distance between a lens center and the center of each opacity area;

FIG. 31 shows an example of a transillumination image of a posterior subcapsular opacity extracted in step S13 in FIG. 3;

FIG. 32 shows examples of an original image of a transillumination image of a lens, a binarized image not subjected to the flat correction process, and a binarized image subjected to the flat correction process;

FIG. 33 shows examples of an original image of a transillumination image of a lens, a binarized image not subjected to the flat correction process, and a binarized image subjected to the flat correction process;

FIG. 34 shows examples of an original image of a transillumination image of a lens, a binarized image not subjected to the flat correction process, and a binarized image subjected to the flat correction process; and

FIG. 35 shows examples of an original image of a transillumination image of a lens, a binarized image not subjected to the flat correction process, and a binarized image subjected to the flat correction process.

## DETAILED DESCRIPTION

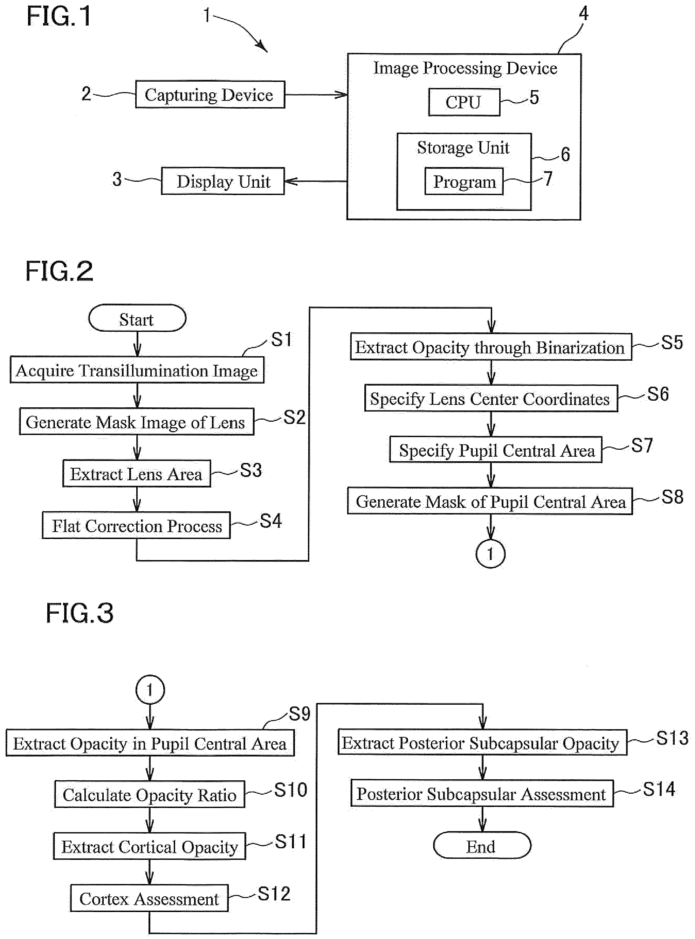

Hereinafter, an embodiment of the present disclosure will be described with reference to the drawings. FIG. 1 shows an image processing system 1 (hereinafter, may be simply referred to as a system) of the present embodiment. The system 1 is a system (lens opacity determination system) that performs determination for an opacity of a lens on the basis of a transillumination image of the lens in order to examine a cataract or decide a cataract surgery method or the like for an eye diagnosed as a cataract.

The system 1 includes a capturing device 2, a display unit 3, and an image processing device 4. The capturing device 2 is, for example, a slit lamp microscope, and is a device for capturing a transillumination image of an eyeball including a lens as an opacity determination target. The capturing device 2 irradiates an eyeball with light, to illuminate a lens by reflection light obtained by the irradiation light being reflected from a retina (fundus) of the eyeball, and captures a frontal image of the eyeball (lens) in the illuminated state.

The capturing device 2 outputs a transillumination image in grayscale, for example. The number of gradation levels of the transillumination image is, for example, 256. In a grayscale image with 256 gradation levels, each pixel value is represented by a value of "0" to "255". A pixel value "0" indicates black, a pixel value "255" indicates white, and pixel values "1" to "254" indicate gray colors different in shade (light-dark). The greater the pixel value of a gray color is, the closer to white the gray color is. In addition, the greater the pixel value is, the higher luminance the pixel value indicates. The transillumination image captured by the capturing device 2 is sent to the image processing device 4.

The display unit 3 displays the transillumination image captured by the capturing device 2, an image processed by the image processing device 4, or the like. The display unit 3 is, for example, a liquid crystal display, but may be another type of display such as a plasma display or an organic EL display.

The image processing device 4 is a part for performing overall control of the system 1. The image processing device 4 has a configuration similar to that of a normal computer, i.e., is composed of a CPU 5, a RAM, a ROM, and the like. The image processing device 4 includes a memory (storage unit 6) storing instructions (program 7), and a processor (CPU 5) which executes the instructions.

The image processing device 4 includes the storage unit 6. The storage unit 6 is a nonvolatile storage unit, and is a non-transitory tangible storage medium which stores, in a non-transitory manner, a program and data that can be read by a computer. The non-transitory tangible storage medium is formed by a semiconductor memory, a magnetic disk, or the like. The storage unit 6 may be configured as a built-in storage unit built in the image processing device 4, or may be configured as an external storage unit externally mounted to the image processing device 4. The storage unit 6 has stored therein various data such as the program 7 for a process to be executed by the CPU 5.

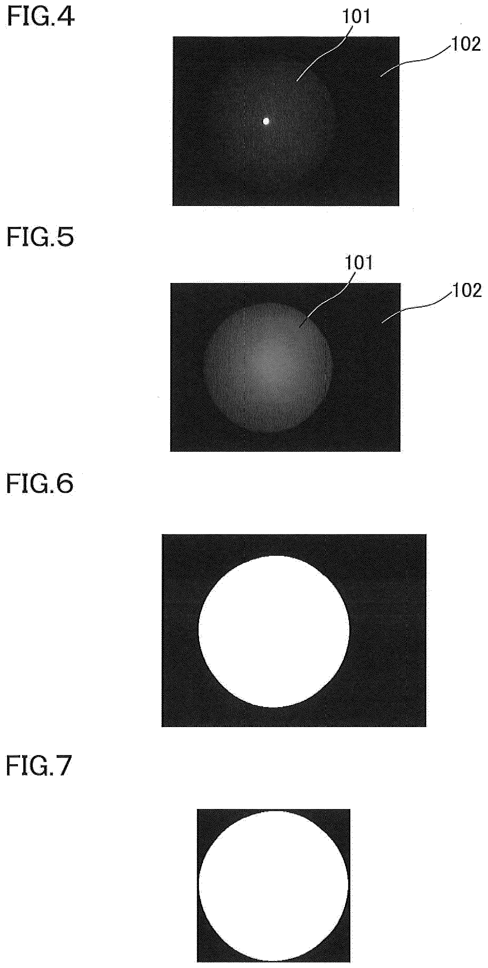

Hereinafter, the details of the process executed by the CPU 5 of the image processing device 4 on the basis of the program 7 will be described. FIG. 2 and FIG. 3 are flowcharts showing an example of the process. The process in FIG. 2 and FIG. 3 is a process for performing determination for an opacity of a lens. When the process in FIG. 2 is started, the CPU 5 acquires a transillumination image captured by the capturing device 2 (S1). FIG. 4 shows an example of the transillumination image acquired in step S 1. The transillumination image (image in FIG. 4) acquired in step S1 includes a circular pupil area (lens area) 101 and an area (iris area) 102 therearound. The CPU 5 executing step S1 corresponds to an acquisition unit in the present disclosure. Step S 1 corresponds to an acquisition step in the present disclosure.

Next, on the basis of the transillumination image acquired in step S 1, a mask image for extracting a lens area is generated (S2). Specifically, first, processing of clarifying the lens area (pupil area) in the transillumination image is performed. Here, for example, a median filter is applied to the original image acquired in step S 1. The median filter is processing of sorting surrounding pixel values in a magnitude order and replacing a target pixel with a median. FIG. 5 shows an image after the median filter has been applied to the image in FIG. 4. As shown in FIG. 5, the boundary between the lens area 101 and the other area 102 has been clarified.

Next, binarization processing is performed to convert each pixel value of the image after the median filter to either white (maximum pixel value (luminance value) = 255) or black (minimum pixel value (luminance value) = 0) using a threshold as a boundary. Specifically, each pixel value is converted to a white pixel value if the pixel value is not smaller than the threshold, and is converted to a black pixel value if the pixel value is smaller than the threshold. The threshold is set so that the lens area has white pixel values and the other area has black pixel values. Here, the threshold is set so that, even in a case where there is an opacity area in the lens area, the opacity area also has white pixel values. FIG. 6 shows an image (binarized image) after binarization processing has been performed on the image in FIG. 5.

Next, an extra black area in the binarized image in FIG. 6 is cut away. FIG. 7 shows an image after the extra black area has been cut away from the image in FIG. 6. The image in FIG. 7 is a mask image in which pixel values of an area corresponding to the lens area are maximum pixel values (= "255"), and pixel values of the other area have minimum pixel values (= "0"). In other words, the image in FIG. 7 is a mask image in which an area corresponding to the lens area is opened and the other area is hidden. The above processing is processing in step S2.

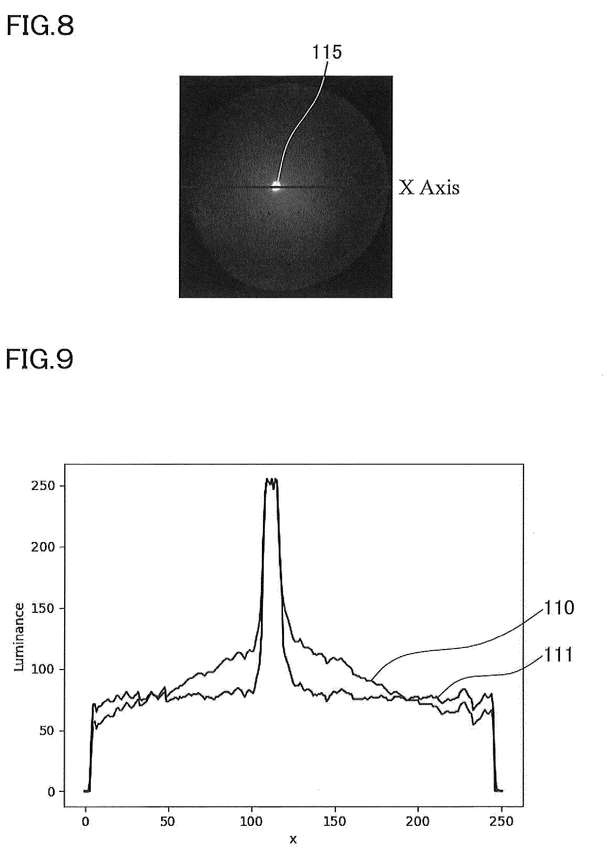

Returning to FIG. 2, next, the mask image generated in step S2 is applied to the original image (transillumination image) acquired in step S1, whereby the lens area in the original image is extracted (S3). In this way, by using the mask image of the lens, an accurate lens area can be extracted. FIG. 8 shows an example of the transillumination image of the lens area extracted in step S3. In addition, a distribution 110 in FIG. 9 represents luminance change along an X axis in the image in FIG. 8. The horizontal axis in FIG. 9 indicates a coordinate (X coordinate) on the X axis, and the vertical axis indicates a luminance value (pixel value). The image in FIG. 8 includes an image 115 indicating spot light of illumination by the capturing device 2. The image 115 is located near the center of the lens area. As shown in FIG. 9, the center of the distribution 110 indicates a local-maximum luminance corresponding to the image 115 shown in FIG. 8. In the distribution 110, the luminance sharply decreases from the center indicating the local-maximum luminance and then gradually (mildly) decreases outward. Thus, there is illumination unevenness in the original image captured by the capturing device 2.

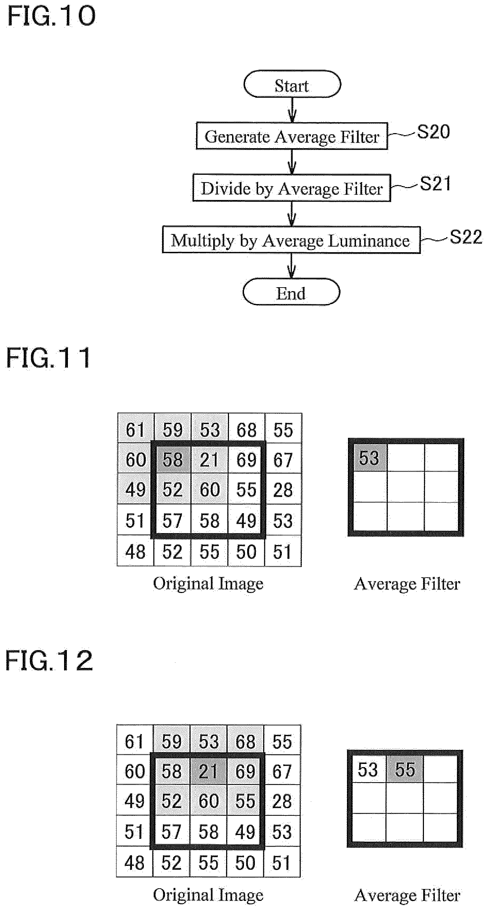

Accordingly, next, correction for reducing illumination unevenness (unevenness of illumination light of the capturing device 2) in the image of the lens area extracted in step S3 is performed (S4). In step S4, a flat correction process is performed to reduce (make close to flat) the gradient of luminance change (the gradient of the luminance that changes outward from the center position indicating the local maximum of the luminance) between image areas due to illumination light radiated to the eyeball by the capturing device 2. As the flat correction process, for example, a process in the flowchart shown in FIG. 10 is performed.

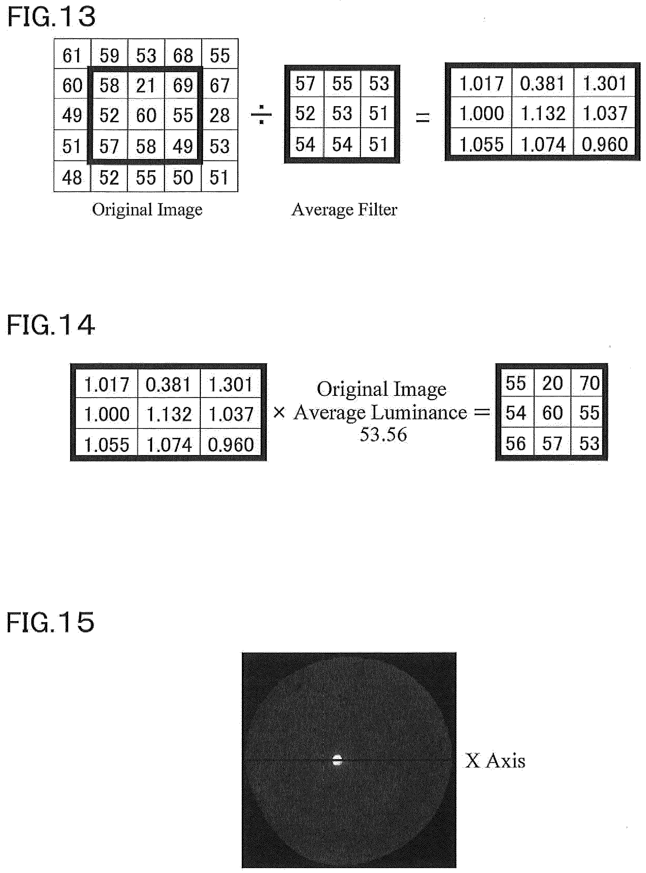

In the process shown in FIG. 10, first, on the basis of the original image (transillumination image) of the lens area extracted in step S3, an average filter is generated (S20). FIG. 11 and FIG. 12 illustrate a method for generating the average filter. In FIG. 11 and FIG. 12, examples of pixel values of the original image are shown, a dense hatched part indicates a target pixel, and a light hatched part indicates pixels around the target pixel. An average value of pixel values of the target pixel and the pixels therearound is calculated, and the average value is set as a value at a position corresponding to the target pixel in the average filter. In the example shown in FIG. 11, an average value of a target pixel value "58" and pixel values therearound is "53", and therefore a value of the average filter corresponding to the target pixel value "58" is "53". In the example shown in FIG. 12, an average value of a target pixel value "21" and pixel values therearound is "55", and therefore a value of the average filter corresponding to the target pixel value "21" is "55". The above processing is performed on each pixel, whereby the average filter is obtained as shown in FIG. 13.

Next, each pixel value of the image extracted in step S3 is divided by the average filter obtained in step S20 (S21). FIG. 13 shows, from the left side, pixel values of the original image, the average filter, and quotient values obtained by dividing the pixel values of the original image by the average filter.

Next, each quotient value (each pixel value after division by the average filter) obtained in step S21 is multiplied by an average luminance (an average value of pixel values of the original image) of the entire original image of the lens area obtained in step S3 (S22). FIG. 14 shows, from the left side, the quotient values (pixel values after division) obtained in step S21, the average luminance of the original image, and corrected pixel values obtained by multiplying these. As shown in the pixel values of the original image in FIG. 13 and the corrected pixel values in FIG. 14, a luminance (pixel value) in an area where luminance change between the target pixel and the pixels therearound is small is corrected so as to converge to a value close to the average luminance of the entire image. On the other hand, a luminance in an area where luminance change between the target pixel and pixels therearound is great (e.g., an area where pixel values are "21" and "69" in the original image in FIG. 13) is corrected to a luminance close to its original image value.

FIG. 15 shows an image after the flat correction process in step S4 (process shown in FIG. 10) has been performed on the image in FIG. 8. A distribution 111 shown in FIG. 9 represents luminance change along the X axis in the image in FIG. 15. In the distribution 111, the gradient of the luminance change outward from the center is smaller than in the distribution 110 before the flat correction process. In addition, luminance information (high-frequency information) at a part where luminance change is great in the distribution 110 before the flat correction process, appears also in the distribution 111 after the flat correction process. Thus, the opacity area of the lens can be inhibited from disappearing or becoming unclear through the flat correction process.

The CPU 5 executing step S4 corresponds to a correction unit in the present disclosure. Step S4 corresponds to a correction step. The CPU 5 executing step S20 corresponds to a filter generation unit. Step S20 corresponds to a filter generation step. The CPU 5 executing step S21 corresponds to a division unit. Step S21 corresponds to a division step. The CPU 5 executing step S22 corresponds to a multiplication unit. Step S22 corresponds to a multiplication step.

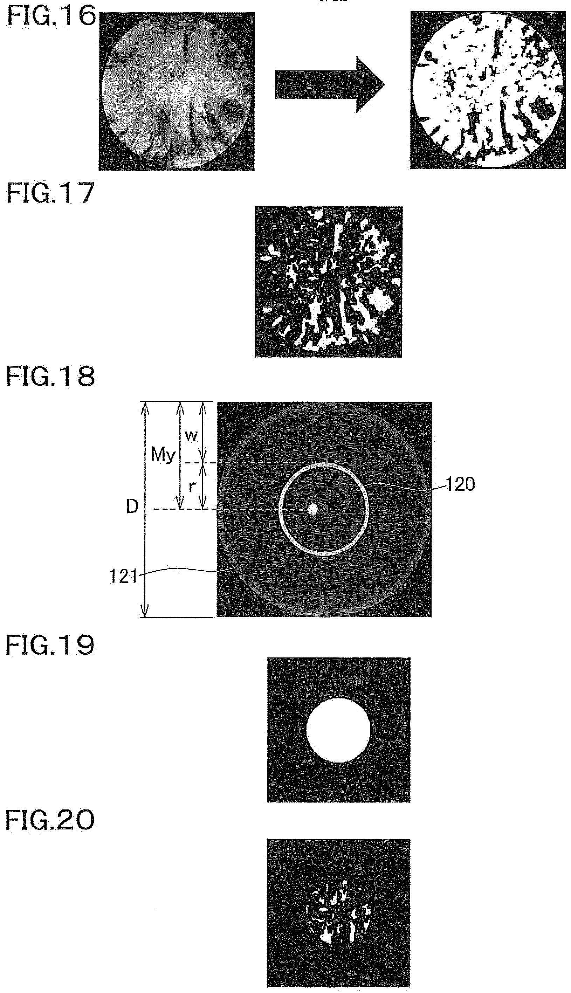

Returning to FIG. 2, next, processing of extracting the opacity area of the lens through binarization from the image after the flat correction process in step S4, is performed (S5). In the opacity area of the lens, the luminance is lower than in an area where there is no opacity. Accordingly, in step S5, the opacity area and the other area are separated from each other on the basis of a difference between the luminance in the opacity area and the luminance in the area where there is no opacity. Specifically, binarization processing is performed to convert each pixel value of the image after the flat correction process to either white (maximum pixel value (luminance value) = 255) or black (minimum pixel value (luminance value) = 0) using a threshold as a boundary. In the binarization processing, each pixel value is converted to a white pixel value if the pixel value is not smaller than the threshold, and is converted to a black pixel value if the pixel value is smaller than the threshold. As the binarization processing, for example, a method of adaptive binarization may be used. The adaptive binarization is a method in which a threshold for separation between white and black is set for each pixel and the threshold is set in accordance with the luminances of the target pixel and pixels therearound. A left image in FIG. 16 shows the image before the binarization processing. A right image in FIG. 16 shows a binarized image obtained by performing the binarization processing on the left image in FIG. 16.

In step S5, further, processing of converting a white area (high-luminance area) in the obtained binarized image to a black area (low-luminance area) and converting a black area (low-luminance area) to a white area (high-luminance area), is performed. That is, pixel values "0" are converted to "255" and pixel values "255" are converted to "0". For example, processing of exclusive disjunction between each pixel value of the mask image obtained in step S2 and each pixel value of the binarized image is performed, whereby conversion can be performed so that the opacity area becomes a high-luminance area and the other area becomes a low-luminance area. FIG. 17 shows an image obtained through the processing of exclusive disjunction between the right image in FIG. 16 and the mask image in FIG. 7. The CPU 5 executing step S5 corresponds to a binarization processing unit in the present disclosure. Step S5 corresponds to a binarization processing step.

Here, a central area and a peripheral area of the transillumination image will be described with reference to FIG. 18. A circle 120 on the inner side in FIG. 18 represents an outer peripheral edge of the central area of the pupil (in other words, a lens area in the pupil) in the transillumination image. That is, the area in the circle 120 represents the central area of the pupil (lens). A circle 121 on the outer side represents an outer peripheral edge of the pupil. The area between the outer circle 121 and the inner circle 120 represents the peripheral area of the pupil (lens). In the peripheral area of the pupil, ease of viewing differs depending on the size of the pupil (the size of the circle 121). On the other hand, the central area of the pupil is always taken as a field of view and therefore is likely to be influenced by a cataract. Accordingly, in steps S6 and S7 in FIG. 2, the central area and the other area (peripheral area) of the pupil (lens) are specified.

Specifically, in step S6, the center coordinates of the lens (pupil) are specified. Here, the lens center coordinates are specified on the basis of moment features of the mask image obtained in step S2. The moment feature is represented by the following Formula 1. Here, x in Formula 1 is an x coordinate of a pixel in the mask image, y in Formula 1 is a y coordinate of a pixel in the mask image, and f(x, y) is such a function that becomes "0" if the pixel value at coordinates (x, y) is "0", and becomes "1" if the pixel value is "255".

$$M_{ij} = \sum_{x} \sum_{y} x^i y^j f(x, y) \quad ----- \text{(Formula 1)}$$

Center coordinates (Mx, My) of the lens are represented by the following Formula 2. The center coordinates (Mx, My) are the center (a position where moments are balanced) of the white area of the lens mask image.

$$(Mx, My) = (M_{10}/M_{00}, M_{01}/M_{00}) \quad ... \text{(Formula 2)}$$

In Formula 2, $M_{00}$ is a value of the moment feature $M_{ij}$ in Formula 1 in a case of i = 0 and j = 0, and is the total number (i.e., area) of pixels in the area (lens area) where the pixel values are "255" in the mask image of the lens. In addition, $M_{10}$ is a value of the moment feature $M_{ij}$ in Formula 1 in a case of i = 1 and j = 0, and $M_{01}$ is a value of the moment feature $M_{ij}$ in Formula 1 in a case of i = 0 and j = 1.

Next, the central area of the pupil is specified (S7). Here, the circle 120 (see FIG. 18) representing the boundary between the central area and the peripheral area of the pupil is specified. The circle 120 is represented by the following Formula 3. In Formula 3, values x, y, Mx, My, and r are each represented by the number of pixels. The values x, y, Mx, and My are each represented by the number of pixels counted from a predetermined position (origin) in the image.

$$(x - Mx)^2 + (y - My)^2 = r^2 \quad ... \text{(Formula 3)}$$

In Formula 3, x is the coordinate in the left-right direction (horizontal direction) of a pixel on the circle 120, y is the coordinate in the up-down direction (vertical direction) of a pixel on the circle 120, Mx is the x coordinate of the lens center obtained in step S6, My is the y coordinate of the lens center obtained in step S6, and r is the radius of the circle 120. The radius r is calculated in light of, for example, WHO grading, as described below.

Examples of kinds of cataracts include a cortical cataract, a nuclear cataract, and a posterior subcapsular cataract. One of assessment methods for cataracts is WHO grading. In WHO grading, it is prescribed that the degree (grade) of the cortical cataract is determined by the ratio of opacity with respect to the entire circumference of the pupil edge. In addition, in WHO grading, it is prescribed that the degree (grade) of the posterior subcapsular cataract is determined by the vertical diameter of opacity present in a 3-mm area at the pupil center. Further, according to the principle of an examination environment in WHO grading, it is prescribed that the mydriasis diameter needs to be not smaller than 6.5 mm, for determination for lens opacity.

Accordingly, for example, the diameter of the circle 120 in FIG. 18 is assumed to be 3.0 mm, the diameter (mydriasis diameter) D of the circle 121 on the outer side is assumed to be 6.5 mm, and then the radius r in Formula 3 is calculated. In a case where the mydriasis diameter D can be calculated accurately, a measurement value thereof may be used as the mydriasis diameter D, instead of 6.5 mm.

Specifically, a length l per pixel is calculated by the following Formula 4. In Formula 4, D is the mydriasis diameter, and is 6.5 mm, here. In addition, N is the number of pixels in the mydriasis diameter D.

$$l = D/N \quad ... \text{(Formula 4)}$$

Then, the number r of pixels of the radius of the pupil central area is calculated by the following Formula 5. In Formula 5, My is the y coordinate of the lens center obtained in step S6. Here, My is represented by the number [px] (px denotes pixel) of pixels counted from the upper end or the lower end of the pupil. In addition, w is the width of the pupil peripheral area (see FIG. 18) and can be calculated by the following Formula 6. Here, D is 6.5 mm and r is 1.5 mm (= 3 mm / 2), so that w is 1.75 mm. In addition, l is the length per pixel obtained by Formula 4. In Formula 5 and Formula 6, r is the radius of the pupil central area, and r in Formula 5 is represented by the number of pixels [px] and r in Formula 6 is represented by a length [mm].

$$r = My - w/l \quad \text{... (Formula 5)}$$

$$w = D/2 - r \quad \text{... (Formula 6)}$$

From the above, the pupil central area represented by Formula 3 is specified. The area other than the pupil central area in the white area of the mask image obtained in step S2 is the peripheral area of the pupil (lens). Returning to FIG. 2, next, a mask image of the pupil central area specified in step S7 is generated (S8). Specifically, an area represented by coordinates (x, y) that satisfy the following Formula 7 is defined as the pupil central area, pixel values in the pupil central area are set at "255" (white), and pixel values in the other area (pupil peripheral area) are set at "0" (black), thus generating a mask image. Here, Mx and My in Formula 7 are obtained by the above Formula 2. The radius r is obtained by the above Formula 5.

$$(x - Mx)^2 + (y - My)^2 \leq r^2 \quad \text{... (Formula 7)}$$

FIG. 19 shows an example of the mask image obtained in step S8. The image in FIG. 19 is a mask image in which the pupil central area is opened and the other area is hidden.
After step S8, the process proceeds to the flowchart in FIG. 3, to extract the opacity area in the pupil central area (S9). Specifically, for example, logical conjunction between the binarized image (image in FIG. 17) of the opacity area in the entire pupil obtained in step S5 and the mask image (image in FIG. 19) obtained in step S8 is calculated. FIG. 20 shows an image obtained by logical conjunction between the image in FIG. 17 and the image in FIG. 19. The image in FIG. 20 is a binarized image in which the opacity area in the pupil central area is represented by white (pixel value "255") and the other area is represented by black (pixel value "0").
Next, an opacity ratio of the lens is calculated (S10). Here, for example, on the basis of the binarized image (image in FIG. 17) of the opacity area in the entire pupil obtained in step S5 and the mask image (image in FIG. 7) of the entire pupil obtained in step S2, a lens opacity ratio (hereinafter, may be referred to as entire opacity ratio) in the entire pupil is calculated. Specifically, the entire opacity ratio is calculated by the following Formula 8. That is, the number of pixels having pixel values "255" in the image in FIG. 17 is divided by the number of pixels having pixel values "255" in the image in FIG. 7.

Entire opacity ratio [%] = Number of white pixels in opacity binarized image of entire pupil/Number of white pixels in mask image of entire pupil

In addition, in step S10, for example, on the basis of the opacity binarized image (image in FIG. 20) of the pupil central area obtained in step S9 and the mask image (image in FIG. 19) of the pupil central area obtained in step S8, a lens opacity ratio (hereinafter, may be referred to as central area opacity ratio) in the pupil central area is calculated. Specifically, the central area opacity ratio is calculated by the following Formula 9. That is, the number of pixels having pixel values "255" in the image in FIG. 20 is divided by the number of pixels having pixel values "255" in the image in FIG. 19.

Central area of opacity ratio [%] = Number of white pixels in opacity binarized image of pupil central area/Numer of white pixels in mask image of pupil central area

The CPU 5 executing step S10 and steps S12 and S14 described later corresponds to an opacity degree determination unit in the present disclosure. Steps S10, S12, and S14 correspond to an opacity degree determination step in the present disclosure.
Next, from the binarized image (image in FIG. 17) of the opacity obtained in step S5, an opacity area (hereinafter, may be referred to as cortical opacity) of the lens cortex is extracted (detected) (S11). Here, the cortical opacity has the following features.

* Spreading radially.
* Appearing from a peripheral area of a lens.

Accordingly, in step S11, the cortical opacity is extracted on the basis of the following algorithm.

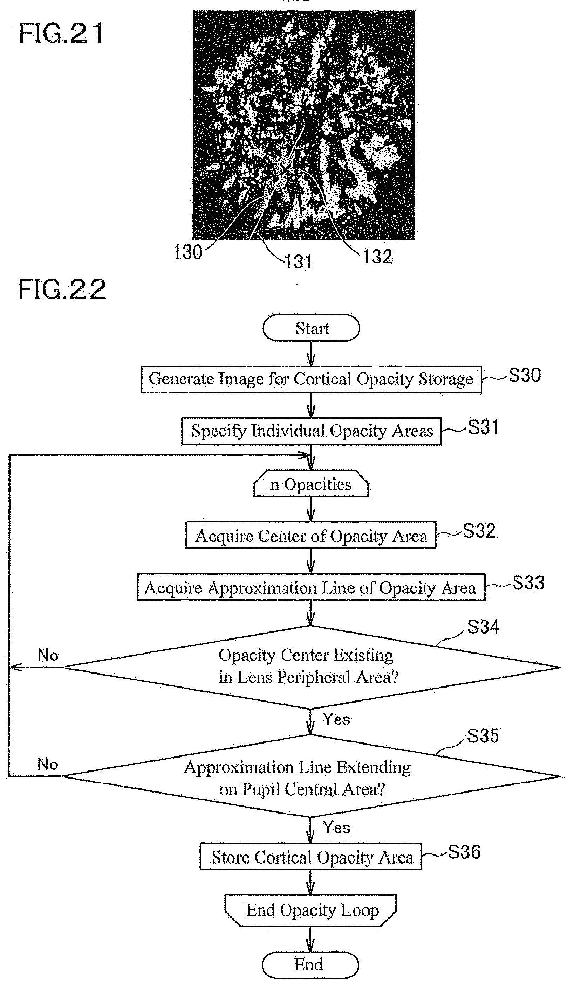

* In FIG. 21, a target opacity area 130 is expressed by a line 131 by a least squares method, and then whether the line 131 extends toward the center of the lens is determined.
* In FIG. 21, whether a center 132 of the target opacity area 130 is located in the peripheral area of the lens is determined.

In step S11, for example, a process shown in FIG. 22 is executed. When the process in FIG. 22 is started, first, an image for cortical opacity storage is generated (prepared) (S30).

Next, individual opacity areas separate from each other and constituting the opacity areas extracted in step S5 are specified (S31). Specifically, the contour of each opacity area extracted in step S5 is detected and each area enclosed by the detected contour is individually specified. Here, it is assumed that n opacity areas are specified in step S31, and then the following steps S32 to S36 are executed for each of the n opacity areas.

That is, with one of the n opacity areas targeted, the coordinates of the center (hereinafter, may be referred to as opacity center) of the target opacity area are acquired (S32). As the opacity center, for example, the coordinates of a position where moments are balanced in the target opacity area are calculated.

Next, an approximation line of the target opacity area is acquired by a least squares method (S33).

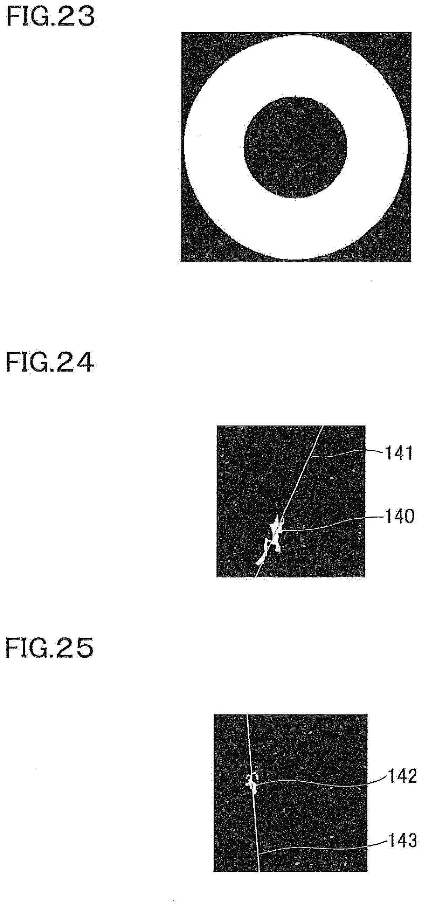

Next, whether the opacity center obtained in step S32 is located in the peripheral area of the lens (in other words, pupil peripheral area) is determined (S34). Specifically, for example, exclusive disjunction between the mask image (image in FIG. 7) of the entire pupil (entire lens) obtained in step S2 and the mask image (mask image in FIG. 19) of the pupil central area (lens central area) obtained in step S8, is calculated, thus obtaining a peripheral area mask image in which the lens peripheral area (pupil peripheral area) has pixel values "255" and the other area has pixel values "0". FIG. 23 shows an example of the peripheral area mask image.

In step S34, whether or not the opacity center obtained in step S32 is located in the white area (lens peripheral area) in the peripheral area mask image is determined. If the opacity center is not located in the lens peripheral area (No in S34), it is determined that the target opacity area at this time does not correspond to a cortical opacity, and the target opacity area is switched to another opacity area, without proceeding to the subsequent step.

On the other hand, if the opacity center is located in the lens peripheral area (Yes in S34), whether the approximation line obtained in step S33 extends on the pupil central area specified in step S7 is determined (S35). Here, for example, whether the approximation line obtained in step S33 overlaps the white area (pupil central area) in the central area mask image (image in FIG. 19) obtained in step S8 is determined. For example, logical conjunction between the approximation line (line having pixel values "255") and the central area mask image (pupil central area having pixel values "255") is calculated, whereby whether or not the approximation line overlaps the pupil central area in the central area mask image can be determined. For example, it is determined that an approximation line 141 of an opacity area 140 shown in FIG. 24 extends on the central area mask image in FIG. 19. Meanwhile, it is determined that an approximation line 143 of an opacity area 142 shown in FIG. 25 does not extend on the central area mask image in FIG. 19.

If the approximation line does not extend on the pupil central area (No in S35), it is determined that the target opacity area at this time does not correspond to a cortical opacity, and the target opacity area is switched to another opacity area, without proceeding to the subsequent step.

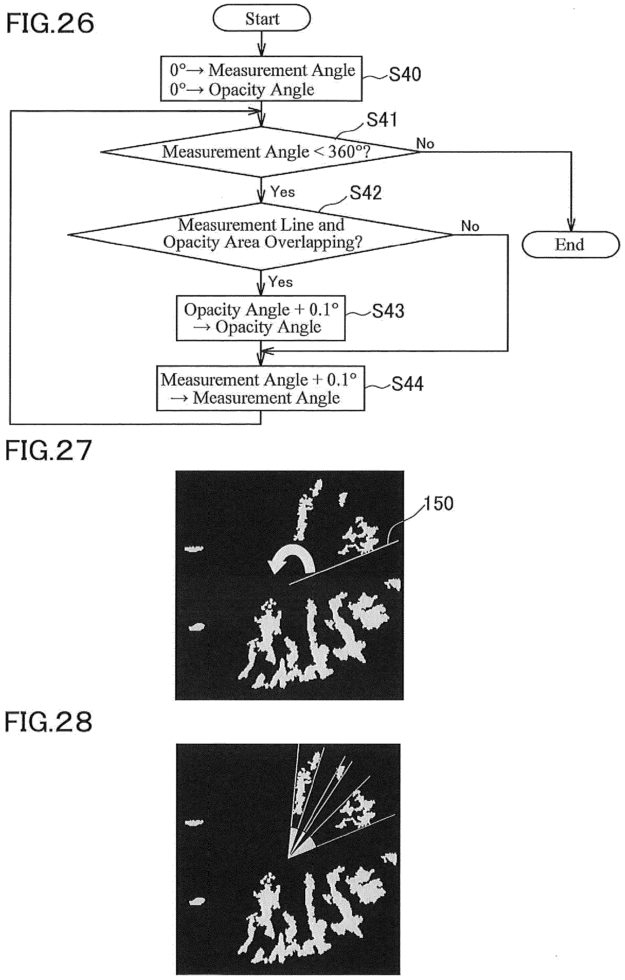

If the approximation line extends on the pupil central area (Yes in S35), it is determined that the target opacity area at this time corresponds to a cortical opacity, and the opacity area is stored in the image generated in step S30 (S36). After steps S32 to S36 have been executed for the n opacity areas, the process in FIG. 22 is ended, thus returning to the process in FIG. 3. In step S 11, a binarized image in which the cortical opacity area is represented as a white area (area having pixel values "255") and the other area is represented as a black area (area having pixel values "0"), is obtained. FIG. 27 shows an example of the binarized image obtained in step S11.

The CPU 5 executing steps S6 to S8 and S11 corresponds to a cortical opacity detection unit in the present disclosure. Steps S6 to S8 and S11 correspond to a cortical opacity detection step. The CPU 5 executing steps S6 and S7 corresponds to an area specifying unit. Steps S6 and S7 correspond to an area specifying step. The CPU 5 executing step S32 corresponds to an opacity center acquisition unit. Step S32 corresponds to an opacity center acquisition step. The CPU 5 executing step S33 corresponds to an approximation line acquisition unit. Step S33 corresponds to an approximation line acquisition step. The CPU 5 executing steps S34 and S35 corresponds to a cortical opacity determination unit. Steps S34 and S35 correspond to a cortical opacity determination step.

Next, the cortical opacity extracted in step S11 is assessed (S12). Here, the size of the cortical opacity, specifically, a total angle of the cortical opacity in the circumferential direction is determined. The CPU 5 executing step S12 corresponds to an opacity size determination unit. Step S12 corresponds to an opacity size determination step. FIG. 26 shows the detailed process of step S 12. When the process in FIG. 26 is started, first, initialization processing is performed to store 0° as each of a measurement angle and an opacity angle which are variables to be used in the subsequent process (S40).

Next, whether or not the present measurement angle is smaller than 360° is determined (S41). If the measurement angle is smaller than 360° (Yes in S41), whether a measurement line which is a line indicating the measurement angle overlaps the area of the cortical opacity extracted in step S11 is determined (S42). The measurement line is a line extending in the radial direction from the lens center specified in step S6, and inclined by the measurement angle with respect to the X axis direction (left-right direction on the image). FIG. 27 shows an example in which a measurement line 150 is set in the image of the cortical opacity obtained in step S11.

If the measurement line overlaps the area of the cortical opacity (Yes in S42), the value of the opacity angle which is a variable is increased by 0.1° (S43). In addition, the value of the measurement angle is increased by 0.1° (S44). Then, the process returns to step S41.

In step S42, if the measurement line does not overlap the area of the cortical opacity (No in S42), the opacity angle is not updated and the value of the measurement angle is increased by 0.1° (S44). Then, the process returns to step S41.

If the measurement angle has reached 360° in step S41 (No in S41), the process in FIG. 26 is ended. Thus, in the process in FIG. 26, the measurement line is set in the image (image in FIG. 27) of the cortical opacity, and while the measurement line is rotated in an arrow direction on a 0.1° basis throughout 360°, the sum of angle ranges where the measurement line overlaps the cortical opacity area is calculated as an opacity angle. FIG. 28 shows a scene during a process when the opacity angle is being calculated.

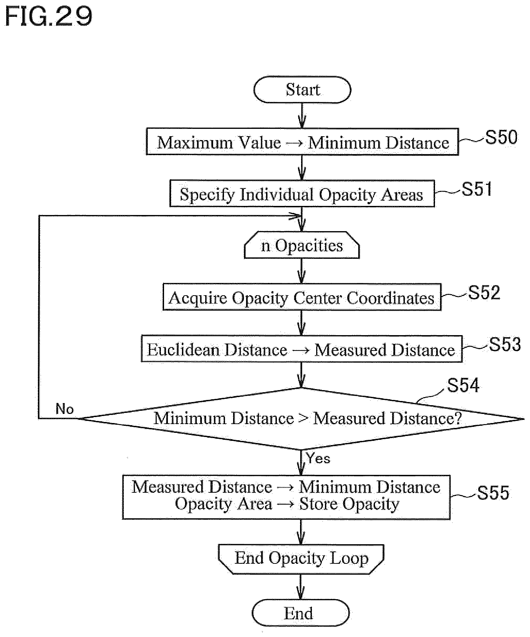

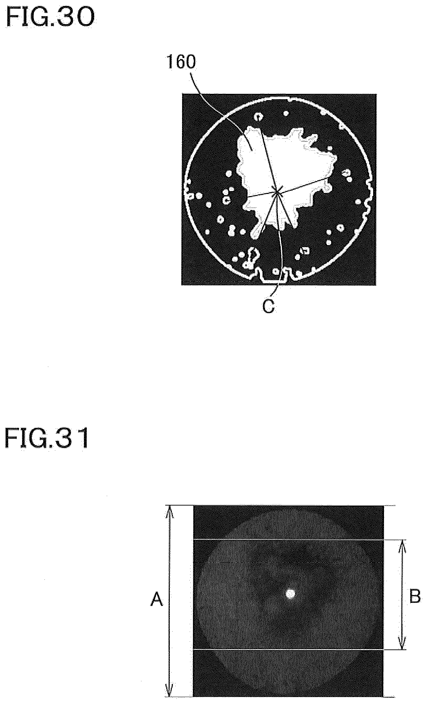

Returning to FIG. 3, next, an opacity area (hereinafter, may be referred to as posterior subcapsular opacity) below the lens posterior capsule is extracted (detected) from the binarized image of the opacity obtained in step S5 (S13). FIG. 30 shows an example of the opacity image obtained in step S5. In step S13, an opacity area 160 closest to a lens center C in the opacity image in FIG. 30 is extracted as a posterior subcapsular opacity. FIG. 29 shows the detailed process of step S13.

When the process in FIG. 29 is started, first, initialization processing is performed to store a maximum value that can be numerically represented, as a minimum distance which is a variable to be used in the subsequent process (S50).

Next, individual opacity areas separate from each other and constituting the opacity areas extracted in step S5 are specified (S51). Specifically, the contour of each opacity area extracted in step S5 is detected and each area enclosed by the detected contour is individually specified. Since each opacity area has already been specified in step S31 in FIG. 22, step S51 may be omitted. Here, it is assumed that n opacity areas are specified in step S51, and then the following steps S52 to S55 are executed for each of the n opacity areas.

That is, with one of the n opacity areas targeted, coordinates P(Px, Py) (hereinafter, may be referred to as opacity center coordinates) of the center of the target opacity area are acquired (S52). As the opacity center coordinates, for example, the coordinates of a position where moments are balanced in the target opacity area are acquired. Since the opacity center coordinates are obtained in step S32 in FIG. 22, the opacity center coordinates obtained in step S32 may be acquired in step S52.

Next, an Euclidean distance d between the opacity center coordinates P(Px, Py) acquired in step S52 and lens center coordinates C(Cx, Cy) is calculated by the following Formula 10 (S53). As the lens center coordinates C(Cx, Cy), the coordinates (Mx, My) obtained in step S6 (the above Formula 2) may be used. The calculated Euclidean distance d is stored as a measured distance which is a variable.

$$d = \sqrt{(C_x - P_x)^2 + (C_y - P_y)^2} \quad \text{------ (Formula 10)}$$

Next, whether or not the measured distance obtained in step S53 is smaller than the minimum distance is determined (S54). If the measured distance is smaller than the minimum distance (Yes in S54), the value of the minimum distance which is a variable is updated to the measured distance (the measured distance is stored as the minimum distance which is a variable), and the opacity area indicating the minimum distance is stored (updated) (S55).

In step S54, if the measured distance is not smaller than the minimum distance (No in S54), the target opacity area is switched to another opacity area, to execute the process from step S52. After steps S52 to S55 are executed for all the opacity areas, the process in FIG. 29 is ended.

As described above, in the process in FIG. 29, the opacity area closest to the lens center C among the n opacity areas is extracted (detected) as a posterior subcapsular opacity. The posterior subcapsular opacity that is extracted in step S13 (process in FIG. 29) may be an opacity area that overlaps the pupil central area specified in step S7 and does not correspond to the cortical opacity extracted in step S11. FIG. 31 shows an example of a transillumination image of the posterior subcapsular opacity extracted in step S13.

The CPU 5 executing step S13 corresponds to a posterior subcapsular opacity detection unit in the present disclosure. Step S13 corresponds to a posterior subcapsular opacity detection step. The CPU 5 executing step S52 corresponds to the opacity center acquisition unit. Step S52 corresponds to the opacity center acquisition step. The CPU 5 executing steps S53 to S55 corresponds to a posterior subcapsular opacity determination unit. Steps S53 to S55 correspond to a posterior subcapsular opacity determination step.

Returning to FIG. 3, next, the posterior subcapsular opacity extracted in step S13 is assessed (S14). Here, the size of the posterior subcapsular opacity, specifically, the width in the vertical direction (up-down direction on the image) of the posterior subcapsular opacity is determined. In the example in FIG. 31, an up-down direction width B of the posterior subcapsular opacity is calculated. For example, the width B is calculated as follows. That is, in light of the examination environment principle in WHO grading, a pupil diameter A is set at 6.5 mm. The length of one pixel is calculated by the following Formula 11. By substituting the length of one pixel calculated by Formula 11 into the following Formula 12, the width B can be calculated.

Lenght of one pixel [mm/px] = 6.5 / Number of pixels within pupil diameter A

$$B \ [\text{mm}] = \text{Number of pixels within width B} \times \text{Length of one pixel} \quad \text{... (Formula 12)}$$

The CPU 5 executing step S14 corresponds to the opacity size determination unit. Step S14 corresponds to the opacity size determination step.

After step S14, the process in FIG. 3 is ended. The CPU 5 may display various information obtained through the process in FIG. 2 and FIG. 3, on the display unit 3. For example, the CPU 5 may display the image (image in FIG. 17 or FIG. 20) of the opacity area extracted in step S5 in FIG. 2 or step S9 in FIG. 3, on the display unit 3. In addition, the CPU 5 may display the opacity ratio obtained in step S10 in FIG. 3, the image (image in FIG. 27) of the cortical opacity extracted in step S 11, the total angle of the cortical opacity obtained in step S12, the image (image in FIG. 31) of the posterior subcapsular opacity extracted in step S13, or the width of the posterior subcapsular opacity obtained in step S14, on the display unit 3.

The CPU 5 may determine which grade applies among a plurality of classified grades of the cortical opacity, on the basis of the total angle of the cortical opacity obtained in step S12, and may display the determination result on the display unit 3. In WHO grading, it is prescribed that the grade of the cortical opacity is determined by the ratio of the cortical opacity with respect to the entire circumference of the pupil edge. Specifically, the grade is "C1" if the ratio is smaller than 1/8, the grade is "C2" if the ratio is smaller than 1/4, the grade is "C3" if the ratio is smaller than 1/2, and the grade is "C4" if the ratio is not smaller than 1/2. The CPU 5 may determine the grade in WHO grading on the basis of the total angle of the cortical opacity. Further, in WHO grading, it is prescribed that, if there is a cortical opacity in a 3-mm area at the pupil center, the cortical opacity is determined as Central + (cen + ). Accordingly, the CPU 5 may determine whether or not the cortical opacity extracted in step S 11 overlaps the pupil central area specified in step S7, and if the cortical opacity overlaps the pupil central area, the CPU 5 may determine the cortical opacity as cen +.

In addition, the CPU 5 may determine which grade applies among a plurality of classified grades of the posterior subcapsular opacity, on the basis of the width of the posterior subcapsular opacity obtained in step S14, and may display the determination result on the display unit 3. In WHO grading, the grade is "P1" if the width of the posterior subcapsular opacity present in a 3-mm area at the pupil center is smaller than 1 mm, the grade is "P2" if the width is not smaller than 1 mm but is smaller than 2 mm, the grade is "P3" if the width is not smaller than 2 mm but is smaller than 3 mm, and the grade is "P4" if the width is not smaller than 3 mm. The CPU 5 may determine the grade in WHO grading on the basis of the width of the posterior subcapsular opacity.

The CPU 5 executing steps S6 to S 14 corresponds to a determination unit in the present disclosure. Steps S6 to S14 correspond to a determination step.

As described above, in the present embodiment, the flat correction process and the binarization processing are performed on a transillumination image of a lens, whereby an image for lens opacity determination that facilitates opacity determination for the lens can be obtained. Thus, erroneous determination for a lens opacity can be prevented.

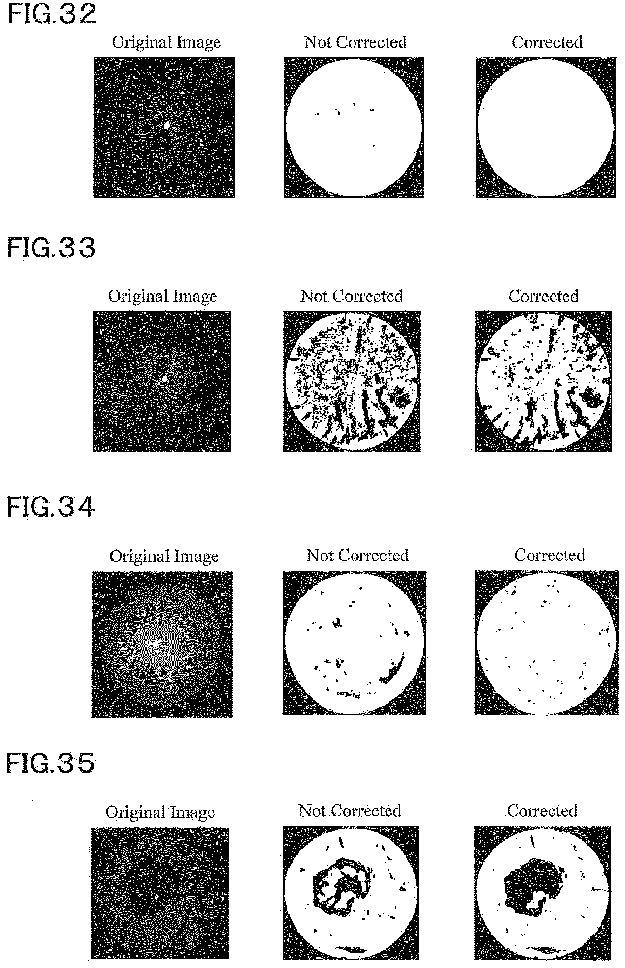

FIG. 32 to FIG. 35 show images obtained from different subjects. FIG. 32 shows images from a healthy subject and FIG. 33 to FIG. 35 show images from cataract patients. Left images in FIG. 32 to FIG. 35 are original images of transillumination images of lenses. Center images in FIG. 32 to FIG. 35 are images obtained by performing binarization processing on the original image without performing the flat correction process in step S4. Right images in FIG. 32 to FIG. 35 are images obtained by performing binarization processing on corrected transillumination images after the flat correction process in step S4 has been performed.

As shown in FIG. 32 to FIG. 35, by performing the flat correction process, a dark illumination area can be inhibited from appearing as a black area in the binarized image, whereby an image that facilitates determination for the position, the shape, the size, and the like of the opacity can be obtained.

Table 1 below shows opacity determination results obtained through the process in FIG. 2 and FIG. 3 with respect to the images in FIG. 32 to FIG. 35. In Table 1, (1) shows an opacity determination result based on the image in FIG. 32, (2) shows an opacity determination result based on the image in FIG. 33, (3) shows an opacity determination result based on the image in FIG. 34, and (4) shows an opacity determination result based on the image in FIG. 35. Grades for a cortex are grades in WHO grading determined on the basis of the angle of a cortex. Grades for a posterior capsule are grades in WHO grading determined on the basis of the width of a posterior capsule. As shown in Table 1, results that the subject of (1) did not correspond to any cataract, the subject of (2) corresponded to a cortical cataract, and the subject of (4) corresponded to a posterior subcapsular cataract, were obtained. In addition, a result that the subject of (3) had a lens opacity was obtained.

[Table 1]

|  | Cortex | Angle [°] | Posterior Capsule | Width [mm] | Opacity in Entirety [%] | Opacity at Center [%] |
|---|---|---|---|---|---|---|
| (1) | C1 | 0 | P1 | 0 | 0 | 0 |
| (2) | C4 | 237.8 | P1 | 0.02 | 20.54 | 14.82 |
| (3) | C1 | 0 | P1 | 0.03 | 0.74 | 0.35 |
| (4) | C1 | 7 | P3 | 2.90 | 18.27 | 58.47 |

As described above, in the present embodiment, the kinds (cortical opacity or posterior subcapsular opacity) of a lens opacity can be automatically determined and the size (degree) of the opacity can be automatically determined. Since determination for the opacity of the lens can be automatically performed, an objective opacity determination result can be obtained.

The present disclosure is not limited to the above embodiment and allows various modifications. For example, although the above embodiment has shown the example in which the kind of an opacity is determined to be a cortical opacity or a posterior subcapsular opacity, determination for another kind of opacity may be performed on the basis of the binarized image after the flat correction process. In addition, although the above embodiment has shown the example in which WHO grading is used for grades of an opacity, opacity grade determination may be performed on the basis of another grading.

In addition, although the above embodiment has shown the example in which opacity determination is automatically performed on the basis of the binarized image after the flat correction process, opacity determination may be performed subjectively by a doctor or the like on the basis of the binarized image.

DESCRIPTION OF THE REFERENCE CHARACTERS

    1 image processing system
    2 capturing device
    3 display unit
    4 image processing device
    5 CPU
    6 storage unit
    7 program

**Claims**

1. An image processing device (4) comprising:

    an acquisition unit (S1, 5) configured to acquire a transillumination image including a lens area (101) and an area (102) therearound, the transillumination image having such a luminance gradient (110) that a luminance gradually decreases outward from a center indicating a local-maximum luminance;
an extraction unit (S2, S3, 5) configured to extract the lens area (101) in the transillumination image;
a correction unit (S4, 5) configured to perform correction so as to reduce the luminance gradient which is illumination unevenness in an image of the lens area extracted by the extraction unit (5); and
a binarization processing unit (S5, 5) configured to binarize pixel values of the corrected image, to obtain a binarized image for lens opacity determination.

2. The image processing device (4) according to claim 1, further comprising a determination unit (S6 to S14, 5) configured to perform determination for an opacity of a lens on the basis of the binarized image.

3. The image processing device (4) according to claim 1 or 2, wherein
the correction unit (S4, 5) includes

a filter generation unit (S20, 5) configured to generate an average filter from an original image of the lens area extracted by the extraction unit,
a division unit (S21, 5) configured to divide each pixel value of the original image by the average filter, and
a multiplication unit (S22, 5) configured to multiply each pixel value of an image that has undergone division by the division unit, by an average luminance of the original image.

4. The image processing device (4) according to claim 2, wherein
the determination unit (S6 to S14, 5) includes a cortical opacity detection unit (S6 to S8, S11, 5) configured to detect a cortical opacity area which is an opacity area of a lens cortex on the basis of the binarized image.

5. The image processing device (4) according to claim 4, wherein
the cortical opacity detection unit (S6 to S8, S11, 5) includes

an area specifying unit (S6, S7, 5) configured to specify a central area of a pupil and a peripheral area of the pupil other than the central area,
an opacity center acquisition unit (S32, 5) configured to acquire an opacity center which is a center of each opacity area represented in the binarized image,
an approximation line acquisition unit (S33, 5) configured to acquire an approximation line of each opacity area represented in the binarized image, and
a cortical opacity determination unit (S34, S35, 5) configured to determine, as the cortical opacity area, the opacity area of which the opacity center is located in the peripheral area and the approximation line overlaps the central area.

6. The image processing device (4) according to claim 4 or 5, wherein
the determination unit (S6 to S14, 5) includes an opacity size determination unit (S12, 5) configured to determine a size of the cortical opacity area detected by the cortical opacity detection unit (S6 to S8, S11, 5).

7. The image processing device (4) according to any one of claims 2 to 6, wherein
the determination unit (S6 to S14, 5) includes a posterior subcapsular opacity detection unit (S13, 5) configured to detect a posterior subcapsular opacity area which is an opacity area below a lens posterior capsule on the basis of the binarized image.

8. The image processing device (4) according to claim 7, wherein
the posterior subcapsular opacity detection unit (S13, 5) includes

an opacity center acquisition unit (S52, 5) configured to acquire an opacity center which is a center of each opacity area represented in the binarized image, and
a posterior subcapsular opacity determination unit (S53 to S55, 5) configured to determine, as the posterior subcapsular opacity area, the opacity area for which a distance between the opacity center and a lens center is smallest among the opacity areas represented in the binarized image.

9. The image processing device (4) according to claim 7 or 8, wherein
the determination unit (S6 to S14, 5) includes an opacity size determination unit (S14, 5) configured to determine a size of the posterior subcapsular opacity area detected by the posterior subcapsular opacity detection unit (S13, 5).

**10.** The image processing device (4) according to any one of claims 2 to 9, wherein
the determination unit (S6 to S14, 5) includes an opacity degree determination unit (S10, S12, S14, 5) configured to determine an opacity ratio of a lens on the basis of the binarized image.

**11.** The image processing device (4) according to claim 10, further comprising:

an area specifying unit (S6, S7, 5) configured to specify a central area of a pupil; and
an opacity extraction unit (S9, 5) configured to extract an opacity area in the central area on the basis of the binarized image and the central area, wherein
the opacity degree determination unit (S10, S12, S14, 5) includes a calculation unit (S10, 5) configured to calculate a lens opacity ratio in the central area on the basis of the central area specified by the area specifying unit (S6, S7, 5) and the opacity area extracted by the opacity extraction unit (S9, 5).

**12.** The image processing device (4) according to any one of claims 1 to 11, further comprising a mask image generation unit (S2, 5) configured to generate a mask image in which the lens area has white pixel values and an area other than the lens area has black pixel values, on the basis of the transillumination image, wherein
the extraction unit (S2, S3, 5) extracts the lens area in the transillumination image on the basis of the mask image.

**13.** The image processing device (4) according to claim 6, wherein
the opacity size determination unit (S12, 5) determines a total angle of the cortical opacity area in a circumferential direction.

**14.** An image processing method comprising:

an acquisition step (S1) of acquiring a transillumination image including a lens area (101) and an area (102) therearound, the transillumination image having such a luminance gradient (110) that a luminance gradually decreases outward from a center indicating a local-maximum luminance;
an extraction step (S2, S3) of extracting the lens area (101) in the transillumination image;
a correction step (S4) of performing correction so as to reduce the luminance gradient which is illumination unevenness in an image of the lens area extracted in the extraction step (S2, S3); and
a binarization processing step (S5) of binarizing pixel values of the corrected image, to obtain a binarized image for lens opacity determination.

**15.** A program (7) configured to cause a computer (5) to execute:

an acquisition step (S 1) of acquiring a transillumination image including a lens area (101) and an area (102) therearound, the transillumination image having such a luminance gradient (110) that a luminance gradually decreases outward from a center indicating a local-maximum luminance;
an extraction step (S2, S3) of extracting the lens area (101) in the transillumination image;
a correction step (S4) of performing correction so as to reduce the luminance gradient which is illumination unevenness in an image of the lens area extracted in the extraction step (S2, S3); and
a binarization processing step (S5) of binarizing pixel values of the corrected image, to obtain a binarized image for lens opacity determination.

## FIG.1

1

4 — Image Processing Device

2 — Capturing Device

CPU — 5

Storage Unit — 6

Program — 7

3 — Display Unit

## FIG.2

Start

Acquire Transillumination Image — S1

Generate Mask Image of Lens — S2

Extract Lens Area — S3

Flat Correction Process — S4

Extract Opacity through Binarization — S5

Specify Lens Center Coordinates — S6

Specify Pupil Central Area — S7

Generate Mask of Pupil Central Area — S8

(1)

## FIG.3

(1)

Extract Opacity in Pupil Central Area — S9

Calculate Opacity Ratio — S10

Extract Cortical Opacity — S11

Cortex Assessment — S12

Extract Posterior Subcapsular Opacity — S13

Posterior Subcapsular Assessment — S14

End

FIG.4

FIG.5

FIG.6

FIG.7

## FIG.8

115

X Axis

## FIG.9

110

111

# FIG.10

```
        ┌──────────┐
        │  Start   │
        └────┬─────┘
             ▼
   ┌─────────────────────────┐
   │  Generate Average Filter │────S20
   └────────────┬────────────┘
                ▼
   ┌─────────────────────────┐
   │  Divide by Average Filter │────S21
   └────────────┬────────────┘
                ▼
   ┌─────────────────────────────┐
   │  Multiply by Average Luminance │────S22
   └──────────────┬──────────────┘
                  ▼
        ┌──────────┐
        │   End    │
        └──────────┘
```

# FIG.11

| 61 | 59 | 53 | 68 | 55 |
|----|----|----|----|----|
| 60 | 58 | 21 | 69 | 67 |
| 49 | 52 | 60 | 55 | 28 |
| 51 | 57 | 58 | 49 | 53 |
| 48 | 52 | 55 | 50 | 51 |

Original Image

| 53 | | |
|----|--|--|
| | | |
| | | |

Average Filter

# FIG.12

| 61 | 59 | 53 | 68 | 55 |
|----|----|----|----|----|
| 60 | 58 | 21 | 69 | 67 |
| 49 | 52 | 60 | 55 | 28 |
| 51 | 57 | 58 | 49 | 53 |
| 48 | 52 | 55 | 50 | 51 |

Original Image

| 53 | 55 | |
|----|----|--|
| | | |
| | | |

Average Filter

# FIG.13

| 61 | 59 | 53 | 68 | 55 |
|----|----|----|----|----|
| 60 | 58 | 21 | 69 | 67 |
| 49 | 52 | 60 | 55 | 28 |
| 51 | 57 | 58 | 49 | 53 |
| 48 | 52 | 55 | 50 | 51 |

$\div$

| 57 | 55 | 53 |
|----|----|----|
| 52 | 53 | 51 |
| 54 | 54 | 51 |

$=$

| 1.017 | 0.381 | 1.301 |
|-------|-------|-------|
| 1.000 | 1.132 | 1.037 |
| 1.055 | 1.074 | 0.960 |

Original Image          Average Filter

# FIG.14

| 1.017 | 0.381 | 1.301 |
|-------|-------|-------|
| 1.000 | 1.132 | 1.037 |
| 1.055 | 1.074 | 0.960 |

Original Image
× Average Luminance =
53.56

| 55 | 20 | 70 |
|----|----|----|
| 54 | 60 | 55 |
| 56 | 57 | 53 |

# FIG.15

X Axis

FIG.16

FIG.17

FIG.18

FIG.19

FIG.20

FIG.21

130   131   132

FIG.22

```
                    ┌─────────┐
                    │  Start  │
                    └────┬────┘
                         ↓
      ┌──────────────────────────────────────┐
      │ Generate Image for Cortical Opacity Storage │──S30
      └──────────────────┬───────────────────┘
                         ↓
          ┌────────────────────────────────┐
          │ Specify Individual Opacity Areas │──S31
          └──────────────┬─────────────────┘
                         ↓
                 ╱ n Opacities ╲
                         ↓
            ┌──────────────────────────────┐
            │ Acquire Center of Opacity Area │──S32
            └──────────────┬───────────────┘
                         ↓
      ┌─────────────────────────────────────────┐
      │ Acquire Approximation Line of Opacity Area │──S33
      └──────────────────┬──────────────────────┘
                         ↓                            S34
      No  ◇─────────────────────────────────────◇
      ←───│    Opacity Center Existing            │
          │   in Lens Peripheral Area?            │
          ◇─────────────────────────────────────◇
                         ↓ Yes
                                                      S35
      No  ◇─────────────────────────────────────◇
      ←───│    Approximation Line Extending       │
          │     on Pupil Central Area?            │
          ◇─────────────────────────────────────◇
                         ↓ Yes
            ┌──────────────────────────────┐
            │   Store Cortical Opacity Area │──S36
            └──────────────┬───────────────┘
                         ↓
                 ╲ End Opacity Loop ╱
                         ↓
                    ┌─────────┐
                    │   End   │
                    └─────────┘
```

FIG.23

FIG.24

FIG.25

**FIG.26**

**FIG.27**

**FIG.28**

# FIG.29

```
                    ┌──────────┐
                    │  Start   │
                    └────┬─────┘
                         ▼
          ┌──────────────────────────────────┐
          │ Maximum Value → Minimum Distance  │──S50
          └────────────────┬─────────────────┘
                           ▼
          ┌──────────────────────────────────┐
          │   Specify Individual Opacity Areas│──S51
          └────────────────┬─────────────────┘
                           ▼
                  ┌──────────────────┐
                  │   n Opacities    │
                  └────────┬─────────┘
                           ▼
          ┌──────────────────────────────────┐
          │  Acquire Opacity Center Coordinates│──S52
          └────────────────┬─────────────────┘
                           ▼
          ┌──────────────────────────────────┐
          │ Euclidean Distance → Measured Distance│──S53
          └────────────────┬─────────────────┘
                           ▼                         S54
         No  ◇───────────────────────────────────────◇
         ┌───  Minimum Distance > Measured Distance?
         │     ◇───────────────────────────────────────◇
         │                  │ Yes
         │                  ▼
         │   ┌──────────────────────────────────┐
         │   │ Measured Distance → Minimum Distance│──S55
         │   │ Opacity Area → Store Opacity       │
         │   └────────────────┬─────────────────┘
         │                    ▼
         │          ┌──────────────────┐
         │          │ End Opacity Loop │
         │          └────────┬─────────┘
         │                   ▼
         │          ┌──────────────────┐
         │          │       End        │
         │          └──────────────────┘
```

FIG.30

FIG.31

## FIG.32

Original Image      Not Corrected      Corrected

## FIG.33

Original Image      Not Corrected      Corrected

## FIG.34

Original Image      Not Corrected      Corrected

## FIG.35

Original Image      Not Corrected      Corrected

Europäisches
Patentamt

European
Patent Office

Office européen
des brevets

# EUROPEAN SEARCH REPORT

Application Number

EP 24 16 8450

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VIVINO M A ET AL: "Quantitative analysis of retroillumination images", EYE, vol. 9, no. 1, 1 January 1995 (1995-01-01), pages 77-84, XP093209362, GB ISSN: 0950-222X, DOI: 10.1038/eye.1995.12 Retrieved from the Internet: URL:https://www.nature.com/articles/eye199512.pdf> | 1-4,6,7, 9-12,14, 15 | INV. G06T7/00 A61B3/117 |
| Y | * abstract, Figs. 1-6, pages 77-83 * | 13 | |
| A | | 5,8 | |
| | ----- | | |
| Y | US 5 632 282 A (HAY S HUTSON [US] ET AL) 27 May 1997 (1997-05-27) | 13 | |
| A | * abstract, Figs. 1-15, claims 1-23, cols. 1-31 * | 1-12,14, 15 | |
| | ----- | | |

TECHNICAL FIELDS
SEARCHED      (IPC)

G06T
A61B

The present search report has been drawn up for all claims

| Place of search | Date of completion of the search | Examiner |
|---|---|---|
| Munich | 26 September 2024 | Borotschnig, Hermann |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

...............................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 16 8450

This annex lists the patent family members relating to the patent documents cited in the above-mentioned European search report.
The members are as contained in the European Patent Office EDP file on
The European Patent Office is in no way liable for these particulars which are merely given for the purpose of information.

26-09-2024

| Patent document cited in search report | Publication date | Patent family member(s) | Publication date |
|---|---|---|---|
| US 5632282 A | 27-05-1997 | NONE | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MAI KITA.** Indication of Cataract Surgery Considering the Age of Patients. *The Journal of The Japanese Society for Cataract Research,* 2017, vol. 29, 56-61